(19) Europäisches Patentamt
European Patent Office
Office européen des brevets

(11) Veröffentlichungsnummer: **0 417 604 B1**

(12) **EUROPÄISCHE PATENTSCHRIFT**

(45) Veröffentlichungstag der Patentschrift: **12.04.95**

(51) Int. Cl.$^6$: **C07F 9/6561**, C07F 9/6574

(21) Anmeldenummer: **90116965.6**

(22) Anmeldetag: **04.09.90**

# (54) Verfahren zur Herstellung von Riboflavin-5'-phosphat bzw. dessen Natriumsalz.

(30) Priorität: **14.09.89 DE 3930668**

(43) Veröffentlichungstag der Anmeldung:
**20.03.91 Patentblatt 91/12**

(45) Bekanntmachung des Hinweises auf die Patenterteilung:
**12.04.95 Patentblatt 95/15**

(84) Benannte Vertragsstaaten:
**CH DE FR GB IT LI NL**

(56) Entgegenhaltungen:

**CHEMICAL ABSTRACTS, Band 83, Nr. 9, 1. September 1975, Seite 695,Zusammenfassung Nr. 79549f, Columbus, Ohio, US;& JP-A-75 25 597 (YAMASA SHOYU CO., LTD) 18-03-1975**

**CHEMICAL ABSTRACTS, Band 83, Nr. 9, 1. September 1975, Seite 695,Zusammenfassung Nr. 89550z, Columbus, Ohio, US;& JP-A-75 25 598 (YAMASA SHOYU CO., LTD) 18-03-1975**

**CHEMICAL ABSTRACTS, Band 83, Nr. 9, 1. September 1975, Seite 695,Zusammenfassung Nr. 79551a, Columbus, Ohio, US;& JP-A 75 25 596 (YAMASA SHOYU CO., LTD) 18-03-1975**

(73) Patentinhaber: **BASF Aktiengesellschaft
Carl-Bosch-Strasse 38
D-67063 Ludwigshafen (DE)**

(72) Erfinder: **Grimmer, Johannes
Duererstrasse 12
D-6700 Ludwigshafen (DE)**
Erfinder: **Kiefer, Hans, Dr.
Im Sandgarten 5
D-6706 Wachenheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft ein verbessertes Verfahren zur Herstellung von Riboflavin-5'-phosphat (5'-Flavinmononucleotid und daher nachfolgend als 5'-FMN bezeichnet) bzw. zur Herstellung des handelsüblichen Mononatriumsalzes von 5'-FMN der Formel I

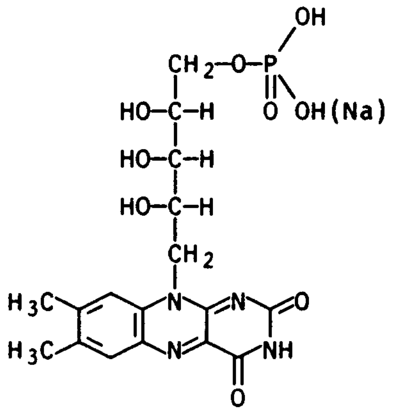

(I).

5'-FMN ist eine Verbindung, die eine wesentliche Rolle als Coenzym in verschiedenen Enzymreaktionen im lebenden Körper spielt und die deshalb in Form ihrer Salze, insbesondere in Form von Natrium-5'-FMN, als Zusatz für Arzneimittel, Nahrungsmittel und Futterstoffe verwendet wird. Natrium-5'-FMN dient auch als Ausgangsmaterial für Flavin-adenin-dinukleotid, das als therapeutisches Mittel gegen Vitamin-$B_2$-Mangel eingesetzt wird.

Das Natrium-5'-FMN wird im allgemeinen durch direktes Umsetzen von Riboflavin mit einem Phosphorylierungsmittel, wie partiell hydrolysiertem Phosphoroxychlorid oder Phosphoroxichlorid in einem organischen Lösungsmittel hergestellt. Die selektive Phosphorylierung ist nicht einfach. So arbeitet man beispielsweise gemäß US-2 610 177 mit einem etwa 20fachen molaren Überschuß an partiell hydrolysiertem Phosphoroxichlorid. Nachteilig an diesem Verfahren ist neben der Verwendung so großer Mengen an Phosphoroxichlorid und der damit verbundenen Umweltbelastung, daß das erhaltene Reaktionsprodukt noch erhebliche Mengen an unumgesetztem Riboflavin, sowie isomere Mono- und Polyphosphate als Nebenprodukte enthält. Daher muß das 5'-FMN einer technisch sehr aufwendigen Reinigungsprozedur unterworfen werden, damit Produkte erhalten werden, die den Reinheitskriterien der US- und der EP-Pharmakopoen entsprechen. Hierzu wird das erhaltene Rohprodukt durch mehrmalige Behandlung mit Ethanolamin oder Morpholin in Form von Monoammoniumsalzen in Lösung gebracht und von nicht umgesetztem und ungelöstem Riboflavin abgetrennt. Diese Reinigungsoperation wird auch in Chemical Engineering, Nov. 1954, Seiten 120 ff. beschrieben.

Gemäß US 2 111 491 erfolgt die Phosphorylierung mit $POCl_3$ in einem sehr großen Überschuß an Pyridin. Nachteilig an diesem Verfahren ist neben dem Einsatz so großer Mengen des giftigen und durch seinen unangenehmen Geruch nicht gern verwendeten Pyridins, daß das Produkt nicht rein und die Aufarbeitung sehr aufwendig ist.

In C.A. 83(1975) 79549f; 79550z und 79551a (JP-OS 25597/1975; JP-OS 25598/1975 bzw. JP-OS 25596(1975) wird die Phosphorylierung von Riboflavin mit einem geringen Überschuß an $POCl_3$ in Lösungsmitteln, wie Tetrahydrofuran, Diethylenglykoldimethylether, Monoethylenglykoldimethylether, Triethylphosphat, 1,2-Dichlorethan oder 1,2-Dibrom-ethan beschrieben. Wie die Vergleichsbeispiele 1 bis 8 zeigen, stellte sich bei der Nacharbeitung der genannten Verfahren heraus, daß das 5'-FMN unter den in loc. cit. angegebenen Reaktionsbedingungen überhaupt nicht oder in nur geringen Mengen gebildet wird. Daß in loc. cit. hohe Ausbeuten an 5'FMN angegeben werden beruht vermutlich auf einem Irrtum, bedingt durch die zu der Zeit noch sehr problematische Analytik dieser Verbindungsklasse.

Allein an den aufwendigen Verfahrensschritten und darüber hinaus dem Einsatz von großen Mengen Phosphoroxichlorid in Bezug auf das zu phosphorylierende Riboflavin (Vitamin $B_2$) ist erkennbar, daß solche Verfahren einen nicht unerheblichen Einfluß auf die Chloridbelastung im Abwasser darstellen können. Auch bei Reinigungsverfahren für Vitamin-$B_2$-Phosphat durch Absorption an einem Celluloseionenaustauscher und Elution mit einem Natriumoxalat/Oxalsäure bzw. Ammoniumformiat/Ameisensäurepuffer (vgl. JA-AS 47/8836 und JA-AS 47/8554) wird das Verfahren nicht wirtschaftlicher und umweltfreundlicher, da bei technischer Nutzung zu große Mengen an Puffersalzen zur Anwendung kommen.

Es war daher die Aufgabe der Erfindung, ein Verfahren zur Herstellung von 5'-FMN bzw. dessen Mononatriumsalz zu entwickeln, bei dem die Nachteile des Standes der Technik vermieden werden, d.h. bei dem 5'-FMN bzw. dessen Natriumsalz auf einfache und umweltschonende Weise hergestellt und in Pharmakopoe-gerechter Reinheit erhalten werden.

Es wurde nun überraschenderweise gefunden, daß man 5'-FMN bzw. Salze des 5'-FMN besonders vorteilhaft erhält, wenn man die Phosphorylierung des Riboflavins mit $POCl_3$ in einem Lacton, insbesondere in γ-Butyrolacton als Lösungsvermittler durchführt. Hierbei bildet sich primär Riboflavin-4'-5'-cyclophosphorsäureesterchlorid der Formel III, das durch Ausfällen mit einem geeigneten Lösungsmittel, wie Methyl-tertiär-Butylether, isoliert werden kann. Die Abtrennung des Esterchlorids der Formel III ist aber nicht notwendig, denn Na-5'-FMN kann durch Phosphorylierung von Riboflavin (Vitamin $B_2$) über die cyclische Zwischenverbindung in einem einzigen Verfahrensschritt in handelsüblicher Form erhalten werden. Die Umsetzung erfolgt im einzelnen nach folgender Gleichung:

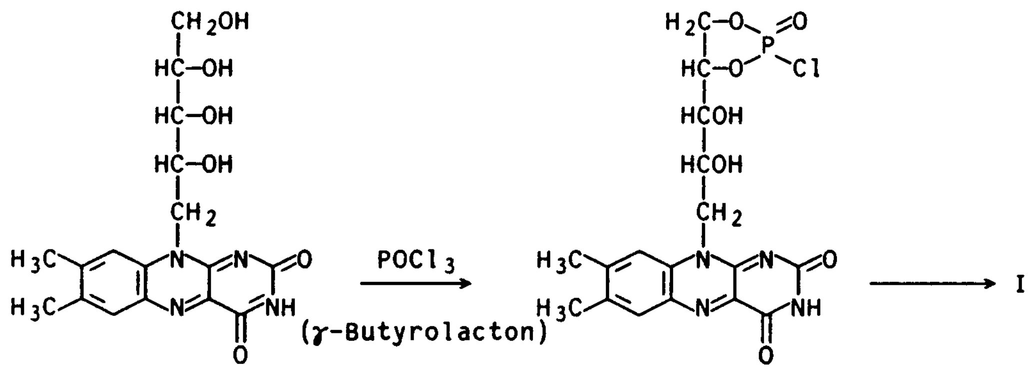


II III

Es war weiterhin überraschend, daß das Lacton bei der Umsetzung von Vitamin $B_2$ mit $POCl_3$ vom Phosphorylierungsmittel nicht angegriffen wird und darüber hinaus auch unter den drastischen Hydrolysebedingungen keinen Schaden erleidet. Es kann nach der Extraktion aus dem wäßrigen Filtrat, aus dem das Na-5'-FMN isoliert wird, wieder als vollwertiges Lösungsmittel für die nächste Phosphorylierung eingesetzt werden.

Gegenstand der Erfindung ist daher ein Verfahren zur Herstellung von Riboflavin-5'-phosphat bzw. dessen Mononatriumsalz durch Umsetzen von Riboflavin mit überschüssigem Phosphoroxichlorid in einem Lösungsmittel, Behandeln des Reaktionsgemisches mit Wasser und gewünschtenfalls anschließende Teilneutralisation des erhaltenen Riboflavinmonophosphats mit wäßriger Natronlauge, das dadurch gekennzeichnet ist, daß man die Umsetzung des Riboflavins mit 1,2 bis 3 mol Phosphoroxichlorid pro mol Riboflavin in einem Lacton, wie γ-Butyrolacton, γ-Decalacton oder γ-Valerolacton durchführt. Von besonderer Bedeutung als Lösungsmittel ist γ-Butyrolacton, da es einerseits sehr vorteilhaft einsetzbar ist und andererseits ein technisch gut zugängliches und im Handel in ausreichender Menge erhältliches Lösungsmittel ist. Darüber hinaus ist γ-Butyrolacton akut wenig toxisch und wird im Körper rasch über γ-Hydroxybuttersäure metabolisiert und so zum größten Teil in Form von $CO_2$ ausgeatmet (vgl. "Toxikolog. Bewertungen, Heft 7, Ausg. 02/89, Seite 1, Berufsgenossenschaft der Chemischen Industrie Heidelberg").

Man verwendet das Lacton im allgemeinen in Mengen von 1 bis 3 l, vorzugsweise etwa 1 bis 1,5 l pro Mol Riboflavin.

Mit besonders guten Ausbeuten gelingt das erfindungsgemäße Verfahren, wenn man das bei der Phosphorylierung erhaltene, das neue Riboflavin-4',5'-cyclophosphorsäureesterchlorids der Formel III enthaltende, Reaktionsgemisch bei Temperaturen zwischen etwa 70 und 90 °C, vorzugsweise 80 und 90 °C mit Wasser behandelt.

Zur Durchführung des erfindungsgemäßen Verfahrens geht man mit Vorteil so vor, daß man das Lacton vorlegt, hierzu Riboflavin und $POCl_3$ addiert, das erhaltene Reaktionsgemisch langsam auf etwa 30 bis 35 °C erwärmt und anschließend bei dieser Temperatur so lange rührt, bis die Umsetzung zu dem Esterchlorid der Formel III beendet ist. Hierzu ist im allgemeinen eine Reaktionszeit von etwa 15 bis 60 Minuten, vorzugsweise etwa 30 bis 60 Minuten notwendig. Nach etwa 30 Minuten wird aus dem Reaktionsgemisch eine dunkle homogene Lösung. Die Reaktionstemperaturen für diese Phosphorylierung können 20 bis 50 °C, vorzugsweise 30 bis 35 °C betragen.

Die anschließende Hydrolyse des Esterchlorids der Formel III zu 5'-FMN kann auf verschiedene Weise diskontinuierlich oder kontinuierlich durchgeführt werden.

Zur diskontinuierlichen Verfahrensweise wird beispielsweise das bei der Phosphorylierung erhaltene Reaktionsgemisch unter Einleiten von Dampf und langsamer Zugabe von vollentsalztem Wasser auf eine Temperatur von 85 bis 90°C erwärmt und anschließend noch etwa 5 bis 20, vorzugsweise etwa 10-15 Minuten auf Temperaturen von 85 bis 95°C gehalten.

Die hierbei verwendete Wassermenge beträgt im allgemeinen 30 bis 50 mol, vorzugsweise 35. bis 40 mol pro mol eingesetztem Riboflavin.

Der Abbruch der unter Hydrolyse einsetzenden Isomerisierung zum 5'-FMN wird durch schnelles Zufügen von weiterem Wasser eingeleitet. Hierzu sind noch einmal etwa 60 bis 100 mol, vorzugsweise 65 bis 80 mol Wasser, bezogen auf eingesetztes Riboflavin notwendig.

Aus dem auf Raumtemperatur abgekühlten Reaktionsgemisch kann dann das 5'-FMN oder nach Versetzen mit wäßriger Natronlauge bis zu einem pH-Wert von etwa 5,5 bis 6 das Natriumsalz des 5'-FMN in an sich bekannter Weise isoliert werden.

Gegenstand der Erfindung ist dementsprechend auch das oben beschriebene Verfahren zur Herstellung von Riboflavin-5'-phosphat bzw. dessen Mononatriumsalz durch Umsetzen von Riboflavin mit 1,2 bis 3 mol $POCl_3$ in einem geeigneten Lacton, bei dem man das bei der Phosphorylierung erhaltene Reaktionsgemisch unter Einleiten von Dampf und langsamer Zugabe von 30 bis 50 mol Wasser pro mol eingesetztem Riboflavin auf eine Temperatur von 85 bis 90°C erwärmt und anschließend noch etwa 5 bis 20 Minuten auf Temperaturen von 85 bis 95°C hält das Reaktionsgemisch mit weiteren 60 bis 100 mol Wasser versetzt, abkühlen läßt und gewünschtenfalls mit wäßriger Natronlauge auf einen pH-Wert zwischen 5,5 und 6 einstellt.

Eine weitere vorteilhafte Variante des erfindungsgemäßen Verfahrens ist dadurch gekennzeichnet, daß man das bei der Phosphorylierung erhaltene Reaktionsgemisch kontinuierlich so in etwa 90 bis 150 mol, 80 bis 100°C heißen Wassers pro mol Riboflavin einträgt, daß die Temperatur des Reaktionsgemisches zwischen etwa 80 und 95°C liegt, das Reaktionsgemisch noch 1 bis 10 Minuten bei dieser Temperatur hält, abkühlen läßt und gewünschtenfalls zur Herstellung des Natriumsalzes mit wäßriger Natronlauge auf einen pH-Wert zwischen 5,5 und 6 einstellt.

Ganz besonders vorteilhaft gestaltet sich diese Variante, wenn man das bei der Phosphorylierung erhaltene Reaktionsgemisch kurzzeitig auf Temperaturen von 40 bis 65°C, vorzugsweise 55 bis 60°C erwärmt bevor man es in das heiße Wasser einträgt.

Eine weitere vorteilhafte Variante des erfindungsgemäßen Verfahrens ist dadurch gekennzeichnet, daß man das bei der Phosphorylierung erhaltene Reaktionsgemisch und Wasser im Verhältnis von 30 bis 150 mol Wasser/mol Riboflavin in einem beheizten Mischgefäß zusammenbringt, nach einer Verweilzeit von 5 bis 15 Minuten bei Temperaturen zwischen 80 und 95°C abkühlen läßt und gewünschtenfalls zur Herstellung des Natriumsalzes mit wäßriger Natronlauge auf einen pH-Wert zwischen 5,5 und 6 einstellt.

Bei der erfindungsgemäßen Herstellung von 5'-FMN bzw. dessen Mononatriumsalz ist darauf zu achten, daß das Riboflavin-4',5'-cyclophosphorsäurechlorid enthaltende Reaktionsgemisch möglichst schnell auf Temperaturen von 80 bis 95°C gelangt und die Temperatur für die angegebene Zeit ohne zwischenzeitliches Absinken der Temperatur bei 80 bis 95°C gehalten wird, da sonst ein Produkt mit unakzeptabel hohem Riboflavingehalt erhalten wird. Weiterhin sollte das Reaktionsgemisch möglichst nicht länger als 10 min auf Temperaturen von 95°C gehalten werden. Bei längerem Erhitzen sollten maximal Temperaturen von 90°C angewendet werden.

Die Umsetzung des Riboflavin-5'-phosphats mit NaOH zu seinem Mononatriumsalz erfolgt im allgemeinen bei Temperaturen von 20 bis 50°C, vorzugsweise 30 bis 40°C.

Gegenstand der Erfindung ist auch das sich insgesamt ergebende elegante Eintropfverfahren zur Herstellung von reinem Riboflavin-5'-phosphat bzw. von seinem Mononatriumsalz, das dadurch gekennzeichnet ist, daß man

A Riboflavin in $\gamma$-Butyrolacton, bei Temperaturen von 20 bis 50°C mit 1,2 bis 3 mol Phosphoroxichlorid pro mol Riboflavin umsetzt,

B das so erhaltene, das neue Riboflavin-4',5'-cyclo-phosphorsäureesterchlorid der Formel III enthaltende Reaktionsgemisch bei Temperaturen zwischen 70 und 90, vorzugsweise 80 und 90°C, mit etwa 30 bis 50 mol Wasser pro mol Esterchlorid behandelt,

C das Reaktionsgemisch 1 bis 15 Minuten auf Temperaturen von 80 bis 95°C, hält,

D anschließend 65 bis 100 mol Wasser pro mol Riboflavin zu dem Reaktionsgemisch addiert und das auskristallisierende Riboflavin-5'-phosphat isoliert, oder gewünschtenfalls

E das gemäß D. erhaltene Reaktionsgemisch bei Temperaturen von 20 bis 50°C, vorzugsweise 30 bis 40°C mit wäßriger NaOH auf einen pH-Wert zwischen 5,5 und 6 einstellt und das auskristallisierende

Mononatriumsalz von Riboflavin-5'-phosphat isoliert.

Das bei dem erfindungsgemäßen Verfahren erhaltene Riboflavin-5'-phosphat enthält im allgemeinen weniger als 6 % Riboflavin und 75 bis 80 % Riboflavin-5'-phosphat und entspricht damit den auf pharmazeutischem Gebiet geltenden Reinheitsanforderungen. Anschließende aufwendige Reinigungsoperationen erübrigen sich. Ein besonderer Vorteil des Verfahrens liegt darin, daß auch Riboflavin minderer Qualität (Roh-Riboflavin z.B.) eingesetzt werden kann, da hierbei Verunreinigungen in der Lösung verbleiben.

Beispiel 1

In einem 1 l Vierhalskolben wurden 180 ml γ-Butyrolacton vorgelegt und nacheinander unter Rühren 54 g Riboflavin (Pharmaqualität = 0,144 mol) und 54 g Phosphoroxichlorid (0,352 mol) eingetragen. Die an der Wandung des Kolbens hängenden Reste wurden mit 36 ml γ-Butyrolacton in das Reaktionsgemisch gespült. Anschließend wurde der Kolbeninhalt langsam auf 30-35 °C erwärmt und 1 Stunde (h) bei dieser Temperatur gerührt. Nach ca. 30 Minuten (min) war das Reaktionsgemisch eine homogene dunkle Lösung. Anschließend wurden rasch (in ca. 1 min) 90 ml vollentsalztes Wasser (VE-Wasser) zugetropft und währenddessen so viel Dampf eingeleitet, daß die Temperatur linear von 35 °C auf maximal 90 °C anstieg. Danach wurde das Reaktionsgemisch 10 min. lang unter Zugabe von weiteren 126 ml VE-Wasser und Einblasen von Dampf auf einer Temperatur von 85 bis 90 °C gehalten. Anschließend wurde durch Zupumpen von weiteren 216 ml VE-Wasser der Abbruch der Isomerisierungsreaktion eingeleitet. Nachdem wurde der Kolbeninhalt durch Kühlen auf 20 bis 25 °C abgekühlt und schließlich mit 315 ml einer 25 %ig wäßrigen Natronlauge versetzt, wobei sich ein pH-Wert von etwa 5,5 einstellte und die Temperatur auf ca. 30 °C anstieg. Anschließend wurde auf Raumtemperatur (RT) abgekühlt, 1 h nachgerührt und über eine G2-Glasfritte das Riboflavin-5'-phosphat-Natrium abgetrennt. Durch Waschen mit ca. 450 ml eines Gemisches aus Methanol und VE-Wasser (60 : 40) und anschließend mit 180 ml reinem Methanol wurde das Wertprodukt von Nebenprodukten befreit. Das gut trockengeblasene, methanolfeuchte Produkt wurde in ca. 350 ml VE-Wasser angeteigt und anschließend sprühgetrocknet.

Die Ausbeute betrug 58 g entsprechend 84 % der Theorie. Gemäß einer Analyse mittels HPLC hatte das Produkt folgende Zusammensetzung:

5 - 7 % Riboflavin-3'-monophosphat
9 - 11 % Riboflavin-4'-monophosphat
76 - 80 % Riboflavin-5'-monophosphat
4 - 5,8 % freies Riboflavin

Beispiel 2

In einem 11 Vierhalskolben wurden 400 ml γ-Butyrolacton vorgelegt und nacheinander unter Rühren 120 g Riboflavin (Pharmaqualität) und 120 g $POCl_3$ in 80 ml γ-Butyrolacton eingetragen. Das erhaltene Gemisch ließ man 1 h bei Temperaturen von 30 bis 35 °C reagieren, erwärmte es anschließend auf 40 °C und pumpte es dann innerhalb von 10 min in 960 ml eines 80 °C heißen vollentsalzten Wassers. Hierbei wurde eine Maximaltemperatur von 87 °C beobachtet. Anschließend wurde noch 6 min bei 87 °C nachgerührt. Dann wurde das Reaktionsgemisch abkühlen lassen, wobei bei etwa 57 °C Riboflavin-5-phosphorsäure ausfiel. Zur Bildung des Natriumsalzes wurde das Reaktionsgemisch bei Temperaturen unterhalb von 40 °C langsam mit 25 %iger wäßriger NaOH-lösung versetzt bis sich ein pH-Wert von 5,5 eingestellt hatte. Anschließend wurde ca. 30 min mit einem Eisbad abgekühlt, der gebildete Niederschlag abgesaugt und zunächst mit 1 l wäßrigem Methanol gewaschen. Der gut trockengesaugte Niederschlag wurde bei 30 °C unter vermindertem Druck bis zur Gewichtskonstanz getrocknet.

Ausbeute: 125,7 g, entsprechend 82 % der Theorie enthaltend gemäß HPLC-Analyse

11,3 % Riboflavin-4-phosphat
71,4 % Riboflavin-5-phosphat
4,5 % Riboflavin und
5,1 % Natrium.

Der optische Drehwert des Produktes (bestimmt nach USP) betrug +38,9 °.

Beispiel 3

Analog Beispiel 2 wurden 120 g Riboflavin (Pharmaqualität) und 120 g $POCl_3$ in 480 ml γ-Butyrolacton 1 h bei Temperaturen von 30 bis 35 °C reagieren lassen, danach das Reaktionsgemisch mit einem Heizbad

von 120 °C auf etwa 60 °C erwärmt und das 60 °C heiße Reaktionsgemisch innerhalb von 10 min in 960 ml 80 °C heißen vollentsalzten Wassers gepumpt, wobei eine maximale Temperatur von 89 °C beobachtet wurde. Anschließend wurde das Reaktionsgemisch noch 6 min bei 89 °C nachgerührt. Aufarbeitung des Reaktionsgemisches analog Beispiel 2 ergab das Mononatriumnsalz von Riboflavin in einer Ausbeute von 134,5 g, entsprechend 88,2 % der Theorie.

Gemäß HPLC-Analyse enthielt dieses Produkt
11,1 % Riboflavin-4-phosphat
70,6 % Riboflavin-5-phosphat
3,7 % Riboflavin und
4,9 % Natrium.

Der optische Drehwert (bestimmt nach USP) betrug +38,6 °.

Beispiel 4

Analog Beispiel 2 wurden 132 g Roh-Riboflavin (Riboflavingehalt 93,5 %) und 120 g $POCl_3$ in 480 ml γ-Butyrolacton 1 h bei 30 bis 35 °C reagieren lassen, danach das Reaktionsgemisch innerhalb von 1 min mit einem 120 °C heißen Heizbad auf eine Temperatur von 67 °C gebracht und das 67 °C heiße Gemisch in 960 ml 80 °C heißen, vollentsalzten Wassers gepumpt, wobei maximale Temperaturen von 89 °C auftraten. Anschließend wurde das Reaktionsgemisch noch 6 min bei 89 °C nachgerührt. Aufarbeitung des Reaktionsgemisches analog Beispiel 2 ergab das Mononatriumnsalz von Riboflavin in einer Ausbeute von 126,6 g.

Gemäß HPLC-Analyse enthielt dieses Produkt
11,6 % Riboflavin-4-phosphat
71,3 % Riboflavin-5-phosphat
4,0 % Riboflavin und
5,1 % Natrium.

Der optische Drehwert (bestimmt nach USP) betrug +39 °.

Beispiel 5

Analog Beispiel 2 wurden 132 g Roh-Riboflavin (Riboflavingehalt 93,5 %) mit 120 g $POCl_3$ in 480 ml γ-Butyrolacton bei 35 bis 37 °C phosphoryliert. Anschließend wurde das Reaktionsgemisch auf 40 bis 45 °C erhitzt und unter Erwärmen langsam mit vollentsalztem Wasser versetzt bis die Temperatur 89 °C erreicht hatte. Die Temperatur blieb etwa 3 min bei 89 °C und wurde dann mittels Wasserbad für 25 min zwischen 68 und 75 °C gehalten. Bei 75 °C wurde der Rest von insgesamt 960 ml Wasser zugetropft. Aufarbeitung des Reaktionsgemisches analog Beispiel 2 ergab das Mononatriumnsalz von Riboflavin in einer Ausbeute von 132,4 g.

Gemäß HPLC-Analyse enthielt dieses Produkt
11,6 % Riboflavin-4-phosphat
71,7 % Riboflavin-5-phosphat
3,9 % Riboflavin und
4,6 % Natrium.

Der optische Drehwert (gemäß USP) betrug 38,9 °.

Vergleichsbeispiele 1 bis 8

In einem 500 ml Vierhals-Rührkolben wurden jeweils 19 g Riboflavin (Gehalt ca. 99 %) in dem aus der Tabelle ersichtlichen Lösungsmittel suspendiert, die Suspension bei einer Temperatur von 25 °C mit der aus der Tabelle ersichtlichen Menge an Phosphoroxychlorid ($POCl_3$) versetzt und anschließend unter den in der Tabelle angegebenen Bedingungen gerührt.

Zur Untersuchung des Reaktionsfortganges wurde dem Reaktionsgemisch jeweils 1 Tropfen entnommen, in etwa 3 ml Wasser von etwa 95 °C gegeben, diese Probe 5 min bei 95 °C gehalten und anschließend mit 6 ml eines Gemisches aus 0,1 m wäßriger Ammoniumformiatlösung und Methanol (im Volumenverhältnis von 4 : 1) als Eluent versetzt und mittels HPLC untersucht.

Tabelle

| Vergleichs-beispiel | gemäß Vorliteratur | Lösungsmittel [ml] | | $POCl_3$ [ml] | Rühren [h/ °C] | Probenahme [nach ...h] | Zusammensetzung des Reaktionsgemisches [%] | | | |
|---|---|---|---|---|---|---|---|---|---|---|
| | | | | | | | 5'-FMN | 4'-FMN | 3'-FMN | Riboflavin |
| 1 | JP-AS 2559/81 (Beispiel 2) | Triethylphosphat | 75 | 12,5 | 20/25 | 1 | 2,8 | 0,7 | 0,8 | 92,8 |
| | | | | | | 3 | 9,5 | 2,4 | 2,7 | 77,2 |
| | | | | | | 20 | 41,1 | 10,3 | 11,6 | 22,8 |
| 2 | JP-AS 2559/81 (Beispiel 2) | Triethylphosphat | 80 | 14 | 3/23-27 | 1 | 4,9 | 1,2 | 1,4 | 87,1 |
| | | | | | | 3 | 12,8 | 3,2 | 1,7 | 70,9 |
| 3 | JP-AS 40158/82 | 1,2-Dichlorethan +1,2-Dibromethan | 35 40 | 14 | 20/25 | 20 | - | - | - | 99,6 |
| 4 | JP-AS 40158/82 | 1,2-Dibromethan | 61 | 14 | 4,5/23 | 4,5 | 0,22 | - | - | 98,1 |
| 5 | JP-AS 40158/82 | 1,2-Dichlorethan | 64 | 14 | 68/24 | 68 | - | - | - | 99,9 |
| 6 | JP-AS 40157/82 | Diethylenglykol-dimethylether | 75 | 14 | 2/25 | 2 | - | - | - | 98,4 |
| | | | | | | 90 | 18,7 | - | - | 71,7 |
| 7 | JP-AS 40157/82 | Diethylenglykol-dimethylether +Tetrahydrofuran | 20 20 | 7 | 3 | 3 | - | - | - | 97,2 |
| | | | | | | 36 | 4,6 | 1,1 | 1,3 | 89,8 |
| 8 | JP-AS 40157/82 | Tetrahydrofuran | 81 | 14 | 18/25 | 18 | - | - | - | 99,2 |

**Patentansprüche**

**1.** Verfahren zur Herstellung von Riboflavin-5'-phosphat bzw. dessen Mononatriumsalz durch Umsetzen von Riboflavin mit überschüssigem Phosphoroxichlorid in einem Lösungsmittel, Hydrolysieren des erhaltenen Reaktionsgemisches durch Behandeln mit Wasser und gewünschtenfalls Teilneutralisation mit wäßriger Natronlauge, dadurch gekennzeichnet, daß man die Umsetzung des Riboflavins mit 1,2 bis

3 mol Phosphoroxichlorid pro mol Riboflavin in einem geeigneten Lacton durchführt.

2. Verfahren gemäß Anspruch 1, dadurch gekennzeichnet, daß man die Umsetzung des Riboflavins mit dem Phosphoroxichlorid in $\gamma$-Butyrolacton durchführt.

3. Verfahren gemäß Anspruch 1, dadurch gekennzeichnet, daß man das erhaltene Reaktionsgemisch bei Temperaturen zwischen etwa 70 und 90 °C mit Wasser behandelt.

4. Verfahren gemäß Anspruch 3, dadurch gekennzeichnet, daß man das bei der Phosphorylierung erhaltene Reaktionsgemisch unter Einleiten von Dampf und langsamer Zugabe von 30 bis 50 mol Wasser pro mol eingesetztem Riboflavin auf eine Temperatur von 85 bis 90 °C erwärmt und anschließend noch etwa 5 bis 20 Minuten auf Temperaturen von 85 bis 95 °C hält, das Reaktionsgemisch mit weiteren 60 bis 100 mol Wasser versetzt, abkühlen läßt und gewünschtenfalls mit wäßriger Natronlauge auf einen pH-Wert zwischen 5,5 und 6 einstellt.

5. Verfahren gemäß Anspruch 3, dadurch gekennzeichnet, daß man das bei der Phosphorylierung erhaltene Reaktionsgemisch kontinuierlich so in etwa 90 bis 150 mol, 80 bis 100 °C heißen Wassers pro mol Riboflavin einträgt, daß die Temperatur des Reaktionsgemisches zwischen etwa 80 und 95 °C liegt, das Reaktionsgemisch 1 bis 10 Minuten bei dieser Temperatur hält, abkühlen läßt und gewünschtenfalls zur Herstellung des Natriumsalzes mit wäßriger Natronlauge auf einen pH-Wert zwischen 5,5 und 6 einstellt.

6. Verfahren gemäß Anspruch 3, dadurch gekennzeichnet, daß man das bei der Phosphorylierung erhaltene Reaktionsgemisch kurzzeitig auf Temperaturen von 40 bis 65 °C erwärmt, die erwärmte Lösung kontinuierlich so in etwa 90 bis 150 mol, 80 bis 100 °C heißen Wassers pro mol Riboflavin einträgt, daß die Temperatur des Reaktionsgemisches zwischen etwa 80 und 95 °C liegt, das Reaktionsgemisch 1 bis 10 Minuten bei dieser Temperatur hält, abkühlen läßt und gewünschtenfalls zur Herstellung des Natriumsalzes mit wäßriger Natronlauge auf einen pH-Wert zwischen 5,5 und 6 einstellt.

7. Verfahren nach Anspruch 3, dadurch gekennzeichnet, daß man das bei der Phosphorylierung erhaltene Reaktionsgemisch und Wasser im Verhältnis von 30 bis 150 mol Wasser/mol Riboflavin in einem beheizten Mischgefäß zusammenbringt, nach einer Verweilzeit von 5 bis 15 min bei Temperaturen zwischen 80 und 95 °C abkühlen läßt und gewünschtenfalls zur Herstellung des Natriumsalzes mit wäßriger Natronlauge auf einen pH-wert zwischen 5,5 und 6 einstellt.

8. Verfahren zur Herstellung von reinem Riboflavin-5'-phosphat bzw. von seinem Mononatriumsalz, dadurch gekennzeichnet, daß man

A Riboflavin in $\gamma$-Butyrolacton bei Temperaturen von 20 bis 50 °C mit 1,2 bis 3 mol Phosphoroxichlorid pro mol Riboflavin umsetzt,

B das so erhaltene, das neue Riboflavin-4',5'-cyclo-phosphorsäureesterchlorid der Formel III

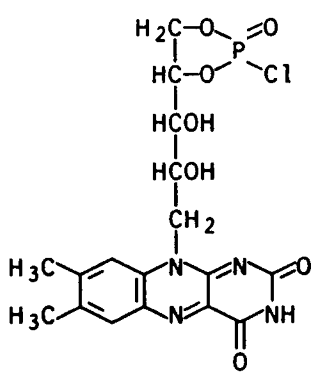

(III)

enthaltende, Reaktionsgemisch bei Temperaturen zwischen 70 und 90 °C mit etwa 30 bis 50 mol Wasser pro mol Esterchlorid behandelt,

C das Reaktionsgemisch 1 bis 15 Minuten auf Temperaturen von 80 bis 95 °C hält,

D anschließend 65 bis 100 mol Wasser pro mol Riboflavin zu dem Reaktionsgemisch addiert und das auskristallisierende Riboflavin-5'-phosphat isoliert, oder gewünschtenfalls
E das gemäß D. erhaltene Reaktionsgemisch bei Temperaturen von 20 bis 40 °C mit wäßriger NaOH auf einen pH-Wert zwischen 5,5 und 6 einstellt und das auskristallisierende Mononatriumsalz von Riboflavin-5'-phosphat isoliert.

## Claims

**1.** A process for preparing riboflavin 5'-phosphate or its monosodium salt by reacting riboflavin with excess phosphorus oxychloride in a solvent, hydrolyzing the resulting reaction mixture by treatment with water and, if desired, partially neutralizing with aqueous sodium hydroxide solution, which comprises reacting the riboflavin with 1.2 to 3 moles of phosphorus oxychloride per mole of riboflavin in a suitable lactone.

**2.** A process as claimed in claim 1, wherein the riboflavin is reacted with the phosphorus oxychloride in $\gamma$-butyrolactone.

**3.** A process as claimed in claim 1, wherein the resulting reaction mixture is treated with water at from about 70 to 90 °C.

**4.** A process as claimed in claim 3, wherein the reaction mixture obtained from the phosphorylation is heated, while passing in steam and slowly adding from 30 to 50 moles of water per mole of riboflavin, to from 85 to 90 °C and then maintained at from 85 to 95 °C for about 5 to 20 minutes, and the reaction mixture is mixed with a further 60 to 100 moles of water, allowed to cool and, if desired, adjusted to a pH of from 5.5 to 6 with aqueous sodium hydroxide solution.

**5.** A process as claimed in claim 3, wherein the reaction mixture obtained from the phosphorylation is continuously introduced into about 90 to 150 moles of water, which is at from 80 to 100 °C, per mole of riboflavin in such a way that the reaction mixture is at from 80 to 95 °C, and the reaction mixture is maintained at this temperature for from 1 to 10 minutes, is allowed to cool and, if desired, adjusted to a pH of from 5.5 to 6 with aqueous sodium hydroxide solution to prepare the sodium salt.

**6.** A process as claimed in claim 3, wherein the reaction mixture obtained from the phosphorylation is briefly heated to from 40 to 65 °C, the heated solution is introduced continuously into about 90 to 150 moles of water at from 80 to 100 °C per mole of riboflavin in such a way that the temperature of the reaction mixture is from 80 to 95 °C, and the reaction mixture is maintained at this temperature for from 1 to 10 minutes, is allowed to cool and, if desired, adjusted to a pH of from 5.5 to 6 with aqueous sodium hydroxide solution to prepare the sodium salt.

**7.** A process as claimed in claim 3, wherein the reaction mixture obtained from the phosphorylation is mixed with from 30 to 150 moles of water per mole of riboflavin in a heated mixing vessel, and after remaining at from 80 to 95 °C for from 5 to 15 minutes it is allowed to cool and, if desired, adjusted to a pH of from 5.5 to 6 with aqueous sodium hydroxide solution to prepare the sodium salt.

**8.** A process for preparing pure riboflavin 5'-phosphate or its monosodium salt, which comprises
A reacting riboflavin with 1.2 to 3 moles of phosphorus oxychloride per mole of riboflavin in $\gamma$-butyrolactone at from 20 to 50 °C,
B treating the resulting reaction mixture which contains the novel cyclic riboflavin 4',5'-phosphochloridate of the formula III

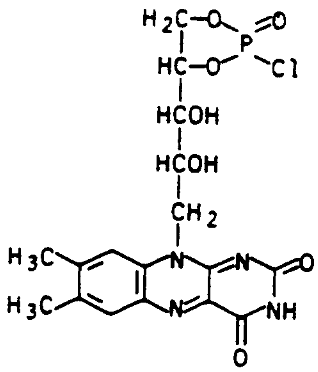

(III)

with about 30 to 50 moles of water per mole of phosphochloridate at from 70 to 90 ° C,

C maintaining the reaction mixture at from 80 to 95 ° C for from 1 to 15 minutes,

D then adding from 65 to 100 moles of water per mole of riboflavin to the reaction mixture and isolating the riboflavin 5'-phosphate which has crystallized out or, if desired,

E adjusting the reaction mixture obtained from D to a pH of from 5.5 to 6 at from 20 to 40 ° C with aqueous NaOH, and isolating the monosodium salt of riboflavin 5'-phosphate which has crystallized out.

## Revendications

1. Procédé de préparation de riboflavine-5'-phosphate ou de son sel monosodique par réaction de riboflavine avec de l'oxychlorure de phosphore en excès dans un solvant, hydrolyse du mélange réactionnel obtenu par traitement par l'eau et, au cas où on le désire, neutralisation partielle avec de la coude aqueuse, caractérisé en ce qu'on met la riboflavine en réaction avec 1,2 a 3 moles d'oxychlorure de phosphore par mole de riboflavine, dans une lactone appropriée,

2. Procédé selon la revendication 1, caractérisé en ce qu'on conduit la réaction de la riboflavine avec l'oxychlorure de phosphore dans de la $\gamma$-butyrolactone.

3. Procédé gelon la revendication 1, caractérisé en ce qu'on traite le mélange réactionnel obtenu par de l'eau a une température comprise entre 70 et 90 ° C environ.

4. Procédé selon la revendication 3, caractérisé en ce qu'on chauffe a une température de 85 a 90 ° C le mélange réactionnel obtenu à la suite de la phosphorylation, avec injection de vapeur d'eau et addition lente de 30 à 50 moles d'eau par mole de riboflavine mise en réaction, puis on le maintient pendant environ 5 à 20 mn encore à une température de 85 à 95 ° C, on additionne de nouveau le mélange réactionnel de 60 à 100 moles d'eau, on le laisse refroidir et, au cas où on le désire, on le règle à un pH compris entre 5,5 et 6 avec de la soude aqueuse.

5. Procédé selon la revendication 3, caractérisé en ce qu'on introduit de façon continue le mélange réactionnel obtenu à la suite de la phosphorylation dans 90 à 150 moles environ d'eau chauffée à 80-100 ° C par mole de riboflavine, de telle sorte que la température du mélange réactionnel se situe entre 80 et 95 ° C environ, on maintient le mélange réactionnel à cette température pendant 1 à 10 mn, on le laisse refroidir et, au cas où on le désire, on le règle à un pH compris entre 5,5 et 6 avec de la soude aqueuse pour la préparation du sel sodique.

6. Procédé selon la revendication 3, caractérisé en ce qu'on chauffe brièvement à une température de 40 à 65 ° C le mélange réactionnel obtenu à la suite de la phosphorylation, on introduit de façon continue la solution chauffée dans 90 à 150 moles environ d'eau chauffée a 80-100 ° C par mole de riboflavine, de telle sorte que la température du mélange réactionnel se situe entre 80 et 95 ° C environ, on maintient le mélange réactionnel à cette température pendant 1 à 10 mn, on le laisse refroidir et, au cas où on le désire, on le règle à un pH compris entre 5,5 et 6 avec de la soude aqueuse, pour la préparation du sel sodique.

**7.** Procédé selon la revendication 3, caractérisé en ce qu'on mélange, dans un récipient mélangeur chauffé, le mélange réactionnel obtenu à la suite de la phosphorylation et de l'eau dans un rapport de 30 à 150 moles d'eau/mole de riboflavine, on le laisse refroidir après une durée de séjour de 5 à 15 mn à une température comprise entre 80 et 95 °C et, au cas où on le désire, on le règle à un pH compris entre 5,5 et 6 avec de la soude aqueuse pour la préparation du sel sodique.

**8.** Procédé de préparation de riboflavine-5'-phosphate pur ou de son sel monosodique, caractérisé en ce que

A l'on fait réagir de la riboflavine dans de la γ-butyrolactone, à une température de 20 à 50 °C, avec 1,2 à 3 moles d'oxychlorure de phosphore par mole de riboflavine,

B on traite le mélange réactionnel ainsi obtenu, contenant le nouveau chlorure d'ester d'acide riboflavine-4',5'-cyclophosphorique de formule III

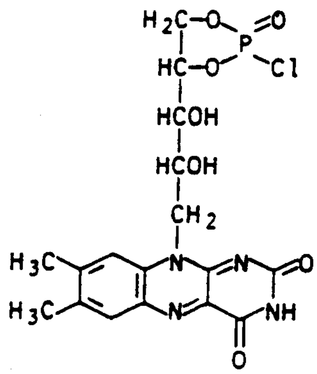

(III)

à une température comprise entre 70 et 90 °C, par 30 à 50 moles environ d'eau par mole de chlorure d'ester,

C on maintient le mélange réactionnel pendant 1 à 15 mn à une température de 80 à 95 °C,

D on ajoute ensuite au mélange réactionnel 65 à 100 moles d'eau par mole de riboflavine et on isole le riboflavine-5'-phosphate qui se sépare à l'état cristallin ou, au cas où on le désire,

E on règle le mélange réactionnel obtenu en D, à une température de 20 à 40 °C, à un pH compris entre 5,5 et 6 avec NaOH aqueux et on isole le sel monosodique de riboflavine-5'-phosphate qui se sépare à l'état cristallin.